# EUROPEAN PATENT SPECIFICATION

(11) **EP 2 477 627 B1**
(45) Date of publication and mention of the grant of the patent: **15.08.2018**
(21) Application number: 10757144.0
(22) Date of filing: 17.09.2010
(51) Int. Cl.: A61K 31/497, A61P 1/10

(54) **USE OF OPIOID RECEPTOR ANTAGONIST FOR GASTROINTESTINAL TRACT DISORDERS**
VERWENDUNG EINES OPIODEN REZEPTORANTAGONISTEN FÜR ERKRANKEN DES MAGEN-DARM-TRAKTS
UTILISATION D'UN ANTAGONISTE DE RÉCEPTEUR D'OPIOÏDE POUR TROUBLES DU TRAJET GASTRO-INTESTINAL

(30) Priority: 18.09.2009 US 243616 P
(43) Date of publication of application: 25.07.2012
(73) Proprietor: Merck Sharp & Dohme Corp., Rahway, NJ 07065 (US)
(72) Inventor: WOODWARD, Richard, M., Phoenixville, PA 19460 (US)
(74) Representative: Jaap, David Robert
(86) International application number: PCT/US2010/049311
(87) International publication number: WO 2011/035142

(56) References cited:
- US-A1- 2006 217 372
- FRIEDMAN J D ET AL: "OPIOID ANTAGONISTS IN THE TREATMENT OF OPIOID-INDUCED CONSTIPATION AND PRURITUS", ANNALS OF PHARMACOTHERAPY, HARVEY WHITNEY BOOKS COMPANY, vol. 35, no. 1, 1 January 2001 (2001-01-01), pages 85-91, XP001095587, ISSN: 1060-0280, DOI: DOI:10.1345/APH.10121

## Description

### 1. BACKGROUND

Pain is the most common reason people seek medical care. Mild to moderate pain is usually treated with acetaminophen and non-steroidal anti-inflammatory drugs. Opioid analgesics, agonists at endogenous µ-(mu), δ- (delta) and/or κ- (kappa) opioid receptors primarily in the central nervous system ("CNS"), are prescribed for moderate to severe acute and chronic pain. Prolonged use of opioid analgesics can result in physical dependence and has side effects such as sedation, mental clouding, nausea, vomiting, constipation and itching.

Opioid-induced constipation ("OIC") and the associated conditions termed opioid induced bowel dysfunction, ("OBD"), are common side-effects in patients who take opioids for more than a few days. Agonists of µ-opioid receptors in the gut and CNS inhibit propulsive motility in the gut, which results in constipation. Other symptoms of OIC or OBD can include incomplete evacuation, abdominal distention, bloating, abdominal discomfort and gastroesophageal reflux. Secondary complications include pseudoobstruction of the bowel, anorexia, nausea, vomiting and interference with oral drug administration and absorption. *See* P. Holzer (2009) Regulatory Peptides 155:11-17.

OIC or OBD can be treated with laxative co-medication, but this approach often has limited efficacy and requires frequent dose adjustment and laxative switching. Other treatments include coadministration of selective opioid receptor antagonists such as naloxone, naltrexone, and N-methylnaltrexone. In order to effectively treat opioid-induced gastrointestinal disorders without reducing analgesia or producing unpleasant and potentially dangerous central opioid withdrawal symptoms, it is critical that threshold pharmacologically-relevant concentrations of opioid antagonists are not established in the CNS. Sub-pharmacological threshold concentrations of opioid antagonists in the CNS have thus far been achieved either by carefully titrating low doses of centrally active antagonists (*e.g*., naloxone), formulating an antagonist with low systemic bioavailability (*e.g*., controlled-release naloxone), or by using opioid antagonists that are restricted to the periphery, *i.e.,* that have restricted access to the CNS (*e.g.,* N-methylnaltrexone).

A fixed-ratio combined formulation of controlled-release naloxone and controlled-release oxycodone (TARGIN®) has been approved in Europe as a way of reducing the severity of OIC.

ENTEREG® (*alvimopan*) has been approved for sale in the United States, and is indicated to accelerate the time to upper and lower gastrointestinal recovery following partial large or small bowel resection surgery with primary anastomosis. This usage is restricted to the short-term treatment of postoperative ileus following bowel resection in hospitalized patients aged 18 years or older. Postoperative ileus is believed to be at least partially caused or exacerbated by exogenous opioid analgesics.

RELISTOR® (n-methylnaltrexone), a product that is administered by subcutaneous injection, has been approved in the United States for the treatment of OIC in patients with advanced medical illness who are receiving palliative care, when response to laxative therapy has not been sufficient.

There is, therefore, a need for more effective, orally-administered treatments for OIC or OBD that do not compromise analgesia or precipitate central opioid withdrawal in patients.

### 2. SUMMARY

The present disclosure provides an amount of 5-(2-methoxy-4-{[2-(tetrahydro-pyran-4-yl)-ethylamino]-methyl}-phenoxy)-pyrazine-2-carboxamide (Compound I) and/or a pharmaceutically acceptable salt thereof for use in treating or preventing OIC or OBD in a subject, which may be an animal or human. The treatment should alleviate the symptoms of OIC or OBD without reducing opioid analgesia or precipitating central opioid withdrawal. The present disclosure further provides the use of 5-(2-methoxy-4- {[2-(tetrahydro-pyran-4-yl)-ethylamino]-methyl} -phenoxy)-pyrazine-2-carboxamide (Compound I) and/or a pharmaceutically acceptable salt thereof for the preparation of a medicament to alleviate the symptoms of OIC or OBD without reducing opioid analgesia or precipitating central opioid withdrawal. The invention described herein is based, in part, on the inventors' surprising discovery that Compound I, an opioid receptor antagonist with comparable potency to known opioid receptor antagonists naltrexone and alvimopan, is sufficiently peripheralized in humans that it will be able to effectively treat OIC or OBD without compromising analgesia or precipitating central opioid withdrawal.

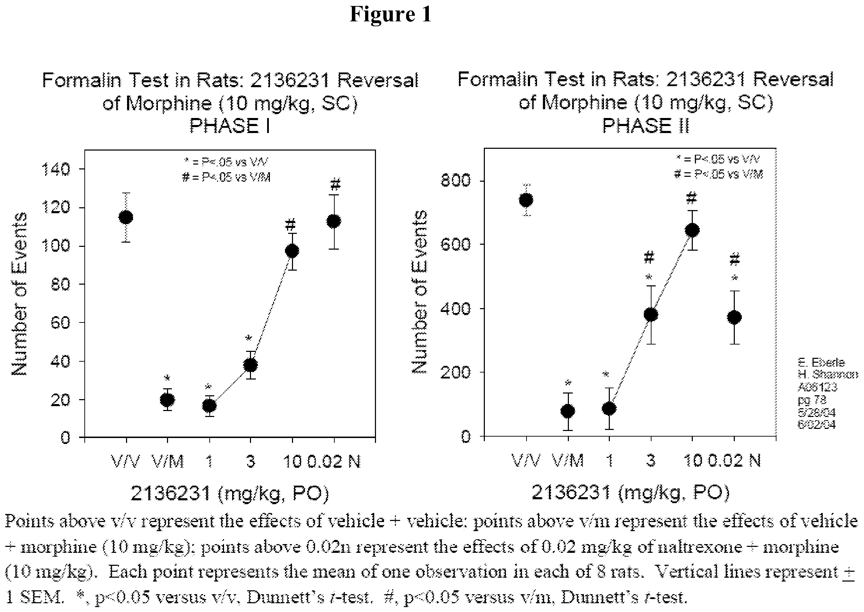

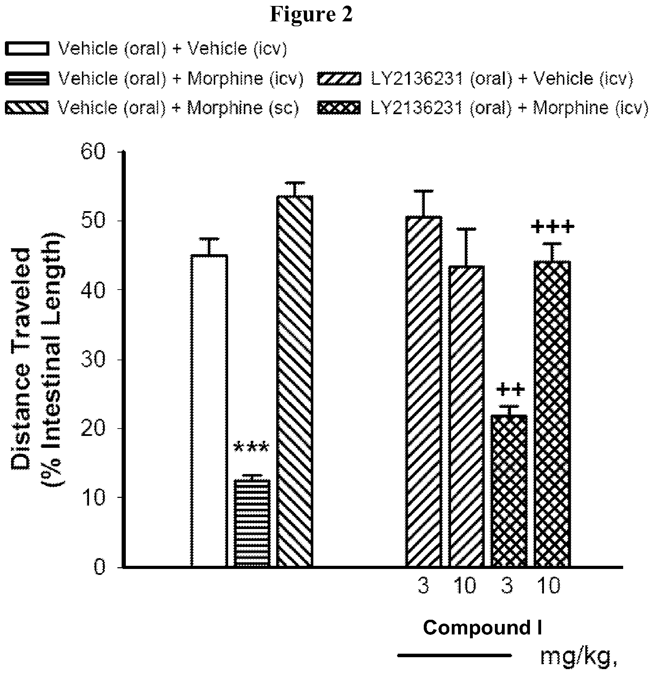

The inventor has studied the results of experiments in known animal models designed to determine the potency and pharmacokinetic properties of opioid receptor antagonists and determined that the data for Compound I were consistent with a potent opioid receptor antagonist that was significantly peripheralized at low doses. In particular, the inventor discerned from the data that Compound I is at least 100 times less potent as an antagonist of opioid receptors in the central nervous system than naltrexone (Example 1, Figure 1). The inventor also noted that Compound I was approximately 20 times more potent at reversing morphine-induced slowing of gut motility in mice when the morphine was administered systemically (*i.e.,* was acting peripherally) than when the morphine was administered directly to the CNS. (Example 1, Figure 2)

The inventor further examined data on the distribution of Compound I in the bodies of rats and mice and noted that there was a much higher concentration (17 times more) in the blood plasma than in the brain. Examination of the distribution in knock-out mice deficient in the P-glycoprotein ("P-gp") transporter revealed that blood-brain penetration increased about 7- to 19-fold. (Example 1) These data indicated to the inventors that Compound I was transported from the CNS by the P-gp transporter, and that the compound was significantly peripheralized in rodents.

Importantly, the inventor further determined an efficacious dose of Compound I for treating OIC or OBD in humans using multiple rationales, described below, based on pharmacokinetic and pharmacological data for Compound I and for the highly peripheralized opioid receptor antagonist alvimopan. In all cases, the calculated effective dose of Compound I for treating OIC or OBD was surprisingly much lower than the dose that affects the CNS, as determined by measured effects on human pharmacodynamic markers for antagonism of opioid receptors in the CNS. Thus, the inventor discovered that Compound I was a potent opioid antagonist that, at very low doses, is sufficiently peripheralized in humans to be useful for treating OIC or OBD and other conditions described herein where selective inhibition of opioid receptors in the periphery is highly desirable, as opposed to inhibition of both peripheral and CNS opioid receptors.

The preliminary results of a Phase I multiple ascending dose clinical study conducted with Compound I in subjects with OIC resulting from chronic opioid therapy indicate that doses as low as 0.10 mg and 0.25 mg were well tolerated and produced the desired pharmacological effects.

### 3. BRIEF DESCRIPTION OF THE FIGURES

Figure 1: Results of rat formalin test. The centrally-acting opioid receptor antagonist naltrexone (given subcutaneously) was found to be more than 100 times more potent than Compound I (2136231, given orally) in reversing opioid analgesia in rats.
Figure 2: Results of oral administration of Compound I on the reversal of the inhibition of gastrointestinal (GI) transit in mice produced by intracerebroventricular (direct CNS) administration of morphine. The minimum effective dose of Compound I required to begin to reverse the inhibitory effect of centrally-administered morphine on GI transit was about 3.0 mg/kg.
Figure 3: Modeled free plasma versus time profile of Compound I given as a 0.15 mg oral dose in humans. "MOR Kᵢ" (solid horizontal line) is the measured Kᵢ of Compound I for the human µ-opioid receptor.
Figure 4: Measured (3 mg and 10 mg) and modeled (0.15 mg and 0.05 mg) free plasma versus time profiles of Compound I given to humans at indicated doses. "MOR Kᵢ" (solid horizontal line) is the measured Kᵢ of Compound I for the human µ-opioid receptor.
Figure 5: (expanding Y-axis of Figure 4): Measured (3 mg and 10 mg) and modeled (0.15 mg and 0.05 mg) free plasma versus time profile of Compound I given to humans at indicated doses. "MOR Kᵢ" (solid horizontal line) is the measured Kᵢ of Compound I for the human µ-opioid receptor.
Figure 6: Alvimopan free plasma versus time profile (data-based modeling) in human OIC patients for a 0.5 mg oral dose, *i.e.,* a dose associated with efficacy in clinical studies.

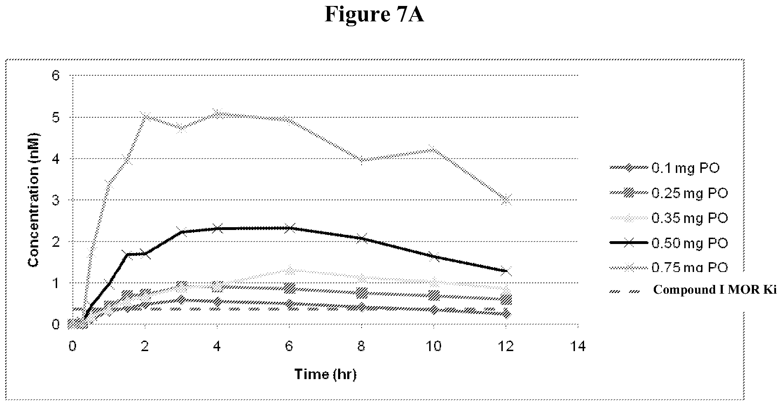

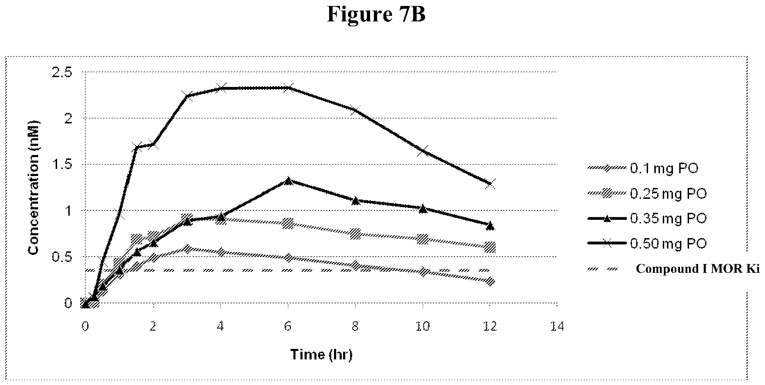

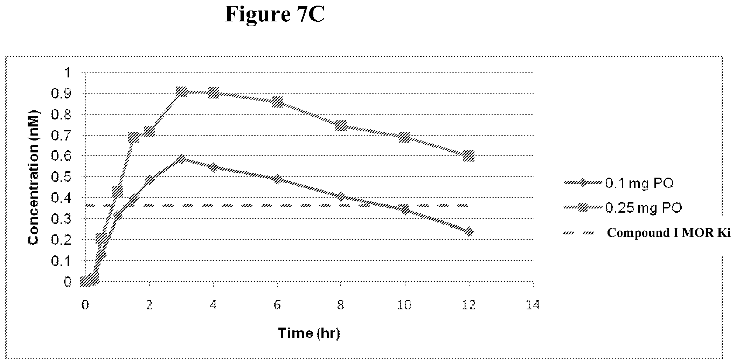

Figures 7A-7C: Profiles of plasma concentration of Compound I versus time measured in a Phase I clinical study of subjects with OIC over the dosing interval of 12 hours. Levels of Compound I in plasma were measured at 15 min, 30 min, 60 min, 90 min, 2 h, 3 h, 4 h, 6 h, 8 h, 10 h and 12 h after the first dose. The measured MOR Kᵢ of Compound I is indicated by a dashed line. Figure 7A shows levels of Compound I in the plasma of subjects for each of the five doses tested. Figure 7B shows levels of Compound I in the plasma of subjects dosed with 0.1 mg, 0.25 mg, 0.35 mg and 0.50 mg of Compound I. Figure 7C shows levels of Compound I in the plasma of subjects dosed with 0.1 mg and 0.25 mg of Compound I.

### 4. DETAILED DESCRIPTION

The present disclosure relates to an effective amount of 5-(2-methoxy-4-{[2-(tetrahydropyran-4-yl)-ethylamino]-methyl}-phenoxy)-pyrazine-2-carboxamide (Compound I) for use in selectively modulating the activity of a µ-, δ- and/or κ-opioid receptor in the periphery, as compared to the CNS, comprising contacting the µ-, δ- and/or κ-opioid receptor with an effective amount of 5-(2-methoxy-4- {[2-(tetrahydro-pyran-4-yl)-ethylamino]-methyl} -phenoxy)-pyrazine-2-carboxamide (Compound I). Compound I has previously been described as a potent centrally active opioid antagonist. *See* U.S. Patent No. 7,381,719. In various embodiments, the present disclosure relates to an effective amount of 5-(2-methoxy-4-{[2-(tetrahydro-pyran-4-yl)-ethylamino]-methyl}-phenoxy)-pyrazine-2-carboxamide (Compound I) for use in treating or preventing a condition associated with the activation of a µ-, δ- and/or κ-opioid receptor in the periphery. In particular embodiments, the disclosure relates to an effective amount of 5-(2-methoxy-4-{[2-(tetrahydropyran-4-yl)-ethylamino]-methyl}-phenoxy)-pyrazine-2-carboxamide (Compound I) for use in treating or preventing a condition associated with the activation of a µ-opioid receptor in the periphery.

In some embodiments, the present disclosure relates to a therapeutically effective amount of Compound I, or a pharmaceutically acceptable salt thereof, for use in treating or preventing a gastrointestinal disorder in a subject. More specifically, the present disclosure relates to a therapeutically effective amount of Compound I, or a pharmaceutically acceptable salt thereof for use in treating or preventing a gastrointestinal disorder in a subject resulting from the use of opioid analgesics without reducing analgesia or producing central opioid withdrawal. In some specific embodiments, the present disclosure relates to a therapeutically effective amount of Compound I, or a pharmaceutically acceptable salt thereof for use in treating or preventing OIC in a subject without reducing analgesia provided by the opioid agonist or producing central opioid withdrawal. In various embodiments, the present disclosure relates to a therapeutically effective amount of Compound I, or a pharmaceutically acceptable salt thereof for use in treating or preventing OBD in a subject without reducing analgesia provided by the opioid agonist or producing central opioid withdrawal.

In various embodiments, the present disclosure relates to the use of Compound I for the preparation of a medicament to selectively modulate the activity of a µ-, δ- and/or κ-opioid receptor in the periphery, as compared to the CNS. In various embodiments, the present disclosure relates to the use of Compound I for the preparation of a medicament for treating or preventing a condition associated with the activation of a µ-, δ- and/or κ-opioid receptor in the periphery. In particular embodiments, the disclosure relates to the use of Compound I for the preparation of a medicament for treating or preventing a condition associated with the activation of a µ-opioid receptor in the periphery.

In certain embodiments, the present disclosure relates to the use of an effective amount of Compound I, or a pharmaceutically acceptable salt thereof, for the preparation of a medicament for the treatment or prevention of a gastrointestinal disorder in a subject. More specifically, the present disclosure relates to the use of Compound I, or a pharmaceutically acceptable salt thereof, for the preparation of a medicament for treating or preventing a gastrointestinal disorder in a subject resulting from the use of opioid analgesics without reducing analgesia or producing central opioid withdrawal. In some specific embodiments, the present disclosure relates to the use of Compound I for the preparation of a medicament for treating or preventing OIC in a subject without reducing analgesia provided by the opioid agonist or producing central opioid withdrawal. In some specific embodiments, the present disclosure relates to the use of Compound I for the preparation of a medicament for treating or preventing OBD in a subject without reducing analgesia provided by the opioid agonist or producing central opioid withdrawal.

The phrases "treatment of," "treating", and the like include the amelioration or cessation of a condition or a symptom thereof, as well as acceleration of recovery from a condition. In one embodiment, treating includes inhibiting, for example, decreasing the overall frequency of episodes of a condition or a symptom thereof.

The phrases "prevention of," "preventing", and the like include the avoidance of the onset of a condition or a symptom thereof.

The term "subject" includes, a human or a non-human animal, such as, *e.g.,* a cow, monkey, baboon, chimpanzee, horse, sheep, pig, chicken, turkey, quail, cat, dog, mouse, rat, rabbit or guinea pig.

The term "opioid" as used herein refers to a class of pain management substances that includes natural or synthetic compounds that bind to and agonize one or more of the µ-, δ- and κ-opioid receptors.

### 4.1. The Compound of Formula I

In certain embodiments, the present disclosure relates to an effective amount of 5-(2-methoxy-4- {[2-(tetrahydro-pyran-4-yl)-ethylamino]-methyl} -phenoxy)-pyrazine-2-carboxamide having the structure shown in formula (I): 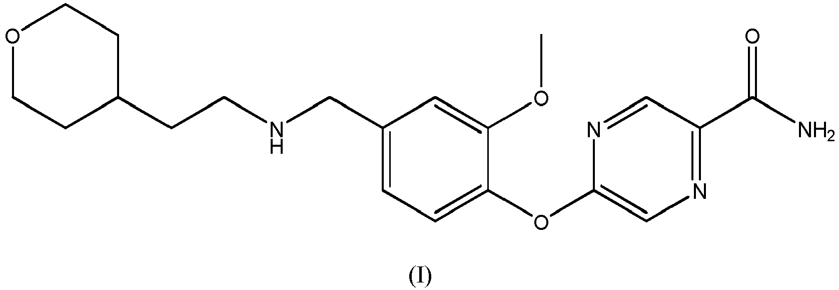 or a pharmaceutically acceptable salt thereof, for use in treating or preventing a condition in a subject associated with activation of peripheral opioid receptors.

The phrase "pharmaceutically acceptable salt," as used herein, is any salt that can be prepared from Compound I including a salt formed from a basic functional group, such as a nitrogen group, of Compound I. Illustrative salts include, but are not limited, to sulfate, citrate, acetate, trifluoroacetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, malate, maleate, gentisinate, fumarate, gluconate, glucuronate, saccharate, formate, benzoate, glutamate, methanesulfonate, ethanesulfonate, benzenesulfonate, *p*-toluenesulfonate, and pamoate (*i.e.*, 1,1'-methylene-bis-(2-hydroxy-3-naphthoate)) salts. One skilled in the art will recognize that acid addition salts of Compound I can be prepared by reaction of the compounds with the appropriate acid via a variety of known methods.

In certain embodiments, the disclosure relates to other forms of Compound I, such as prodrugs, radiolabeled forms, solvates (*e.g.,* hydrates, or addition compounds that contain acid in excess of the number of their basic nitrogen atoms due to hydrogen bonding), amorphous solid forms and crystalline solid forms.

Compound I can be obtained by any method known in the art. In certain embodiments, Compound I can be synthesized using the starting materials 5-(4-formyl-2-methoxyphenoxy)pyrazine-2-carboxamide and 2-(tetrahydropyran-4-yl)ethylamine and the protocol and reagents set forth in Example 720 of U.S. Patent No. 7,381,719.

### 4.2. Therapeutic Uses

Due to its activity as a peripherally-selective opioid receptor antagonist, Compound I is advantageously useful in human and veterinary medicine to treat or prevent a condition that can be alleviated by selective antagonism of an opioid receptor in the periphery. Compound I can be administered to any subject requiring inhibition of a peripheral opioid receptor. In some embodiments, Compound I is advantageously useful to treat or prevent a condition that can be alleviated by selective antagonism of a peripheral µ-opioid receptor. In various embodiments, Compound I is useful for treating or preventing a condition selected from, but not limited to, postoperative ileus, postoperative nausea and vomiting, opioid-induced nausea and vomiting, opioid-induced respiratory depression, opioid-induced constipation, opioid-induced bowel dysfunction, chronic idiopathic constipation, constipation-predominant irritable bowel syndrome, intestinal pseudoobstruction, delayed gastric emptying, enteral feeding intolerance, narcotic ileus, obstipation, acceleration of gastrointestinal recovery following surgery and opioid-induced bloating. In some preferred embodiments, Compound I is useful for treating or preventing a gastrointestinal motility disorder in a subject. In certain embodiments, the subject is a human.

In some embodiments, due to its potent and selective antagonism of peripheral opioid receptors as compared to central opioid receptors, Compound I is useful for treating or preventing a condition in a subject resulting from the use of opioids without reducing their analgesic effect or producing central opioid withdrawal. In particular embodiments, Compound I is useful for treating or preventing OIC without reducing the analgesic effect of the opioid or producing central opioid withdrawal. In other embodiments, Compound I is useful for treating or preventing OBD without reducing the analgesic effect of the opioid or producing central opioid withdrawal.

### 4.3. Therapeutic Compositions and Methods of Administration

When administered to a subject, Compound I can be administered as a component of a composition that comprises a pharmaceutically acceptable carrier or excipient. The compositions comprising Compound I can be administered orally. The compositions can also be administered by any other convenient route, for example, by infusion or bolus injection, by absorption through epithelial or mucocutaneous linings (*e.g*., oral, rectal, and intestinal mucosa, etc.) and can be administered together with a second therapeutically active agent (*e.g.,* a laxative, a stool softener or an opioid analgesic). Administration can be systemic or local. Various delivery systems are known, *e.g*., encapsulation in liposomes, microparticles, microcapsules, multiparticulates, capsules, etc., and can be used to administer Compound I.

Methods of administration include, intradermal, intramuscular, intraperitoneal, parenteral, intravenous, subcutaneous, intranasal, oral, sublingual, transdermal, rectal, by inhalation, or topical. In most instances, administration will result in the release of Compound I into the bloodstream. In preferred embodiments, administration will not result in release in the CNS at pharmacologically relevant concentrations.

Pulmonary administration can also be employed, *e.g.,* by use of an inhaler or nebulizer, and formulation with an aerosolizing agent, or via perfusion in a fluorocarbon or synthetic pulmonary surfactant. In certain embodiments, Compound I can be formulated as a suppository, with traditional binders and excipients such as triglycerides.

When Compound I is incorporated for parenteral administration by injection (*e.g.*, continuous infusion or bolus injection), the formulation for parenteral administration can be in the form of a suspension, solution, emulsion in an oily or aqueous vehicle, and such formulations can further comprise pharmaceutically necessary additives such as one or more stabilizing agents, suspending agents, dispersing agents, and the like. Compound I can also be in the form of a powder for reconstitution as an injectable formulation.

Compound I can be delivered in a vesicle, in particular a liposome (*see* Langer, Science 249:1527-1533 (1990); and Treat et al., Liposomes in the Therapy of Infectious Disease and Cancer 317-327 and 353-365 (1989)).

Compound I can be delivered in a controlled-release system or sustained-release system (*see, e.g.,* Goodson, "Dental Applications" (pp. 115-138) in Medical Applications of Controlled Release, Vol. 2, Applications and Evaluation, R.S. Langer and D.L. Wise eds., CRC Press (1984)). Other controlled or sustained-release systems discussed in the review by Langer, Science 249:1527-1533 (1990) can be used. A pump can be used (Langer, Science 249:1527-1533 (1990); Sefton, CRC Crit. Ref. Biomed. Eng. 14:201 (1987); Buchwald et al., Surgery 88:507 (1980); and Saudek et al., N. Engl. J. Med. 321:574 (1989)). Alternatively, polymeric materials can be used (see Medical Applications of Controlled Release (Langer and Wise eds., 1974); Controlled Drug Bioavailability, Drug Product Design and Performance (Smolen and Ball eds., 1984); Ranger and Peppas, J. Macromol. Sci. Rev. Macromol. Chem. 23:61 (1983); Levy et al., Science 228:190 (1985); During et al., Ann. Neurol. 25:351 (1989); and Howard et al., J. Neurosurg. 71:105 (1989)).

The compositions can optionally comprise a suitable amount of a pharmaceutically acceptable excipient so as to provide the form for proper administration to the subject. Such a pharmaceutical excipient can be a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. The pharmaceutically acceptable excipient can be sterile when administered to a subject. Water is a particularly useful excipient when the compound of formula (I) is administered intravenously. Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, American Pharmaceutical Association (1986).

The compositions comprising Compound I can take the form of solutions, suspensions, emulsions, tablets, pills, pellets, capsules, capsules containing liquids, powders, sustained release formulations, suppositories, emulsions, aerosols, sprays, suspensions, sublingual disintegrating dosage forms, or any other form suitable for use. The composition can be in the form of a capsule (see, e.g., U.S. Patent No. 5,698,155). Other examples of suitable pharmaceutical excipients are described in Remington's Pharmaceutical Sciences 1447-1676 (Alfonso R. Gennaro ed., 19th ed. 1995).

Compound I can be formulated in accordance with routine procedures as a composition adapted for oral administration to humans. Compound I to be orally delivered can be in the form of tablets, capsules, gelcaps, caplets, lozenges, sublingual disintegrating solid dosage forms, aqueous or oily solutions, suspensions, granules, powders, emulsions, syrups, or elixirs, for example. When Compound I is incorporated into oral tablets, such tablets can be compressed tablets, tablet triturates (e.g., powdered or crushed tablets), enteric-coated tablets, sugar-coated tablets, film-coated tablets, multiply compressed tablets or multiply layered tablets. Techniques and compositions for making solid oral dosage forms are described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16th ed., Mack Publishing, Easton, PA 1980).

Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents, and the like. Techniques and compositions for making liquid oral dosage forms are described in Pharmaceutical Dosage Forms: Disperse Systems, (Lieberman, Rieger and Banker, eds.) published by Marcel Dekker, Inc.

An orally administered composition can contain one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions can include standard excipients such as mannitol, lactose, starch, magnesium stearate, sodium saccharin, cellulose, and magnesium carbonate. In one embodiment, the excipients are of pharmaceutical grade.

Orally administered compositions can be formulated to release the active ingredient in the lower gastrointestinal tract, and particularly, in the colon. Such dosage forms for release of the active ingredient in the colon can include polymers having pH-dependent solubility that dissolve at the pH of the colon but not at the pH of the small intestine; polymers that have a slow or pH-dependent rate of swelling, dissolution or erosion so that the dosage form dissolves in the colon rather than in the small intestine; polymers that are degraded by microbial enzymes in the colon; or polymers that form layers that are broken by increased luminal pressure caused by peristaltic waves. Compositions include hydroxypropylmethyl cellulose capsules coated with materials that provide for release in the colon, such as those disclosed in U.S. Patent No. 7,094,425. Still other compositions for release in the colon are disclosed in U.S. Patent No. 6,368,629 and include dosage forms that are coated with an acid-labile coating that is dissolved by enterobacteria.

When Compound I is to be injected parenterally, it can be, *e.g.,* in the form of an isotonic sterile solution. Alternatively, when Compound I is to be inhaled, it can be formulated into a dry aerosol or can be formulated into an aqueous or partially aqueous solution.

Compound I can be formulated for intravenous administration. Typically, compositions for intravenous administration comprise sterile isotonic aqueous buffer. Where necessary, the compositions can also include a solubilizing agent. A composition for intravenous administration can optionally include a local anesthetic such as benzocaine or prilocaine to lessen pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where Compound I is to be administered by infusion, it can be dispensed, for example, with an infusion bottle containing sterile pharmaceutical grade water or saline. Where Compound I is administered by injection, an ampule of sterile water for injection or saline can be provided so that the ingredients can be mixed prior to administration.

Compound I can be in a dosage form together with an opioid analgesic. Opioid analgesics that can be formulated with Compound I include, alfentanil, buprenorphine, butorphanol, codeine, dezocine, dihydrocodeine, fentanyl, hydrocodone, hydromorphone, levorphanol, meperidine (pethidine), methadone, morphine, nalbuphine, oxycodone, oxymorphone, pentazocine, propiram, propoxyphene, sufentanil and tramadol. Other opioid analgesics for inclusion in a dosage form comprising Compound I will be readily ascertained by the skilled artisan.

### 4.4. Therapeutic Dosages

The amount of Compound I that is effective for treating OIC or OBD in humans was extrapolated from pharmacokinetic and pharmacological data using calculations based on multiple rationales - for example, (i) comparison of pharmacokinetic and pharmacological data for Compound I with pharmacokinetic and pharmacological data with the peripheralized opioid receptor antagonist alvimopan from human efficacy studies in OIC, or (ii) calculations based on the dose of Compound I that would produce maximum free plasma concentrations (Cₘₐₓ) equal to or greater than the Kᵢ of Compound I for the µ-opioid receptor, as set forth below in Example 2. Using multiple rationales, the whole body dose of Compound I that is effective in treating OIC or OBD without antagonizing µ-opioid receptors in the CNS, and thus reducing opioid analgesia or producing central opioid withdrawal, was calculated to be approximately 0.1 mg/day, to be administered either QD or BID. The amount of Compound I effective for treating OIC or OBD that was extrapolated from pharmacokinetic and pharmacological data as described above was confirmed in Phase I clinical studies of subjects with OIC to be approximately 0.1 mg/day.

The dose margin, *i.e.,* the fold difference between the calculated effective dose of Compound I for treating OIC or OBD and the mean effective dose determined by experimentation to be required to reverse the effects of morphine in the CNS, can be from about 25 to about 83. *See* Example 2. Accordingly, at low doses required for treating OIC or OBD, Compound I was found to be highly peripheralized, and thus unlikely to compromise opioid analgesia or to precipitate central opioid withdrawal.

Suitable effective dosage amounts for treating OIC or OBD or other conditions described herein in an average-size human male (*e.g*., weighing about 70 kg) range from about 0.01 mg to about 1.5 mg whole body dose per day. In certain embodiments, suitable effective dosage amounts range from about 0.02 mg to about 1.25 mg whole body dose per day, such as from about 0.03 mg to about 1.0 mg whole body dose per day, such as from about 0.04 mg to about 0.75 mg whole body dose per day, such as from about 0.05 to about 0.5 mg whole body dose per day.

In certain embodiments, suitable effective dosage amounts range from about 0.1 to about 1 mg whole body dose per day. In various embodiments, suitable effective dosage amounts range from about 0.1 to about 0.75 mg whole body dose per day, such as from about 0.1 to about 0.5 mg whole body dose per day, such as from about 0.1 to about 0.35 mg whole body dose per day, such as from about 0.1 to about 0.25 mg whole body dose per day.

In various embodiments, suitable effective dosage amounts range from about 0.01 mg to about 1.0 mg whole body dose per day, such as from about 0.02 mg to about 0.9 mg whole body dose per day, such as from about 0.03 mg to about 0.8 mg whole body dose per day, such as from about 0.04 mg to about 0.7 mg whole body dose per day, such as from about 0.05 mg to about 0.6 mg whole body dose per day, such as from about 0.06 mg to about 0.5 mg whole body dose per day, such as from about 0.07 mg to about 0.4 mg whole body dose per day, such as from about 0.08 mg to about 0.3 mg whole body dose per day.

In still other embodiments, suitable effective dosage amounts for treating OIC or OBD or other conditions described herein in an average-size human male (*e.g*., weighing about 70 kg) range from about 0.05 mg to about 0.5 mg whole body dose per day, such as from about 0.06 mg to about 0.25 mg whole body dose per day, such as from about 0.07 mg to about 0.2 mg whole body dose per day, such as from about 0.08 mg to about 0.15 mg whole body dose per day.

In various embodiments, suitable effective dosage amounts for treating OIC or OBD or other conditions described herein in an average-size human male (*e.g*., weighing about 70 kg) range from about 0.01 mg to about 0.5 mg whole body dose BID, such as from about 0.02 mg to about 0.45 mg whole body dose BID, such as from about 0.03 mg to about 0.4 mg whole body dose BID, such as from about 0.04 mg to about 0.35 mg whole body dose BID, such as from about 0.05 mg to about 0.3 mg whole body dose BID, such as from about 0.06 mg to about 0.25 mg whole body dose BID, such as from about 0.07 mg to about 0.2 mg whole body dose BID, such as from about 0.08 mg to about 0.15 mg whole body dose BID.

Suitable effective dosage amounts for treating OIC or OBD or other conditions described herein range from about 0.0001 mg/kg/day to about 0.02 mg/kg/day. In certain embodiments, suitable effective dosage amounts range from about 0.0003 mg/kg/day to about 0.017 mg/kg/day, such as from about 0.0004 mg/kg/day to about 0.014 mg/kg/day, such as from about 0.0005 mg/kg/day to about 0.01 mg/kg/day, such as from about 0.0007 mg/kg/day to about 0.007 mg/kg/day.

In various embodiments, suitable effective dosage amounts range from about 0.0001 mg/kg/day to about 0.014 mg/kg/day, such as from about 0.0003 mg/kg/day to about 0.013 mg/kg/day, such as from about 0.0004 mg/kg/day to about 0.011 mg/kg/day, such as from about 0.0006 mg/kg/day to about 0.01 mg/kg/day, such as from about 0.0007 mg/kg/day to about 0.009 mg/kg/day, such as from about 0.0009 mg/kg/day to about 0.007 mg/kg/day, such as from about 0.001 mg/kg/day to about 0.006 mg/kg/day, such as from about 0.001 mg/kg/day to about 0.004 mg/kg/day.

In still other embodiments, suitable effective dosage amounts for treating OIC or OBD or other conditions described herein range from about 0.0007 mg/kg/day to about 0.007 mg/kg/day, such as from about 0.0009 mg/kg/day to about 0.004 mg/kg/day, such as from about 0.001 mg/kg/day to about 0.003 mg/kg/day, such as from about 0.001 mg/kg/day mg to about 0.002 mg/kg/day.

In various embodiments, suitable effective dosage amounts for treating OIC or OBD or other conditions described herein range from about 0.00015 mg/kg to about 0.007 mg/kg BID, such as from about 0.0003 mg/kg to about 0.006 mg/kg BID, such as from about 0.0004 mg/kg to about 0.004 mg/kg BID, such as from about 0.0006 mg/kg to about 0.005 mg/kg BID, such as from about 0.0007 mg/kg to about 0.004 mg/kg BID, such as from about 0.0009 mg/kg to about 0.004 mg/kg BID, such as from about 0.001 mg/kg to about 0.003 mg/kg BID, such as from about 0.001 mg/kg to about 0.002 mg/kg BID.

In certain embodiments, Compound I is administered to a subject that is sensitized to its effects and that thereby requires a lower dose for efficacy in treating a condition as described herein. In some embodiments, the lower effective dose produces a free plasma concentration of Compound I that is about 0.75Kᵢ. In other embodiments, the lower effective dose produces a free plasma concentration that is about 0.5Kᵢ. In still other embodiments, the lower effective dose produces a free plasma concentration that is about 0.33Kᵢ. In still other embodiments, the lower effective dose produces a free plasma concentration of Compound I that is about 0.75K_{b}. In yet other embodiments, the lower effective dose produces a free plasma concentration that is about 0.5K_{b}. In various embodiments, the lower effective dose produces a plasma concentration that is about 0.33K_{b}.

It will be understood that the amount of Compound I that is effective for the treatment or prevention of a condition described herein can be determined by standard clinical techniques. In addition, *in vitro* and/or *in vivo* assays can optionally be employed to help identify optimal dosage ranges. The precise dose to be employed will also depend on, *e.g.,* the route of administration, formulation, the seriousness of the condition, and level of narcotic analgesia, and can be decided according to the judgment of a practitioner and/or each subject's circumstances. In other examples thereof, variations will necessarily occur depending upon the weight and physical condition (*e.g*., hepatic and renal function, the severity of an underlying disease) of the subject being treated, the affliction to be treated, the severity of the symptoms, the frequency of the dosage interval, and the presence of any deleterious side-effects, among other things.

It will be understood that, in some embodiments, the dose per day can be administered as a single dose. In other embodiments, the dose per day can be administered as a divided dose. In still other embodiments, the dose of Compound I can be administered as needed (PRN).

In certain embodiments, Compound I is administered prior to or subsequent to administration of an opioid analgesic. In other embodiments, Compound I is administered at the same time as administration of an opioid analgesic. In some embodiments, Compound I and the opioid analgesic can be administered by the same route of administration (*e.g*., orally). In other embodiments, Compound I and the opioid analgesic are administered by different routes of administration. In certain embodiments where Compound I and the opioid analgesic are administered together, the opioid analgesic and Compound I are administered in a single dosage form (*e.g.,* in a capsule, co-formulated tablet or disintegrating sublingual solid dosage form).

### 5. EXAMPLES

### Example 1: Pharmacokinetics and Pharmacology of Compound I in Non-Clinical Models

### Potency of Compound I in the CNS compared to naltrexone

Rats were injected with the irritant formalin and with 10 mg/kg of morphine subcutaneously. The amount of analgesia was determined by the reduction in the number of times the rat licked or paid attention to the site of formalin injection (events). Rats were dosed either with 0.02 mg/kg naltrexone (subcutaneous dose) or 1, 3 or 10 mg/kg Compound I (oral dose) to determine the ability of the compounds to reverse the analgesic effect of morphine. Naltrexone was found to be approximately 100 times more potent than Compound I in reversing the effects of morphine in the CNS of rats. (Figure 1) Naltrexone and Compound I have comparable affinity for µ-opioid receptors. Separate experiments indicate that Compound I has high oral bioavailability in rat. The reversal of morphine-induced analgesia by opioid receptor antagonists in the formalin test is believed to be primarily a centrally-mediated effect. The discrepancy in potency suggests that Compound I has markedly lower levels of penetration into the CNS than naltrexone.

### Effect of Compound I in the CNS and in the periphery

Mice were either dosed with10 µg/kg morphine intracerebroventricularly or with 1 mg/kg morphine subcutaneously. The degree of gastrointestinal transit of charcoal meal in the mice was measured in the absence of Compound I and in the presence of varying oral doses of Compound I in order to determine the ability of the compound to reverse the inhibition of gastrointestinal (GI) transit induced by morphine. The minimum effective dose of Compound I required to reverse GI transit inhibition in mice dosed with morphine directly into the CNS was determined to be about 3.0 mg/kg oral dose. (Figure 2) In contrast, the effective dose of Compound I required to reverse GI transit inhibition in mice dosed with morphine systemically was determined to be about 0.16 mg/kg oral dose. Thus, Compound I was about 20 times more potent in reversing the peripheral effects of morphine in slowing GI transit as compared to the central effects of morphine in slowing GI transit. This suggests that Compound I was about 20 times more effective in antagonizing opioid receptors in the periphery than in the CNS.

### Partitioning of Compound I in the plasma and brains of wild-type and P-glycoprotein transporter knockout mice

Wild-type or P-glycoprotein (P-gp) knockout mice were dosed with 2.5 mg/kg of Compound I intravenously. The mice were sacrificed and the amounts of Compound I in the blood and in the brain were assayed using standard techniques. The results of these experiments showed that 17 times more Compound I was found in the blood plasma than in the brain of wild type mice. Moreover, blood-brain barrier penetration of Compound I increased 7- to 19-fold in knockout mice that were deficient in the P-gp transporter. The results of these experiments indicated that Compound I was actively transported out of the CNS by P-gp, resulting in a steady-state exclusion of Compound I from the CNS, and that Compound I was therefore signifcantly peripheralized in mice.

### Example 2: Calculated Effective Dose of Compound I For Treating OIC or OBD in Humans and Determination of Sufficiency of Peripheralization Not to Compromise Central Analgesia or Produce Central Opioid Withdrawal

The non-clinical animal studies described above indicate that Compound I exhibits a degree of peripheralization in mice and rats and further begin to delineate the major mechanism(s) by which Compound I is excluded from the CNS, *i.e.,* Compound I is a substrate for the efflux transporter P-gp. However, the key questions are whether these data have relevance to humans, and what impact they have on the therapeutic utility of Compound I for treating conditions in humans.

The primary issue is whether Compound I is sufficiently peripheralized *in humans* to make it viable for treating conditions, in particular OIC or OBD, which rely on selective antagonism of opioid receptors in the periphery as compared to antagonism of opioid receptors in the CNS, which would compromise centrally-mediated analgesia and produce unpleasant and potentially dangerous central opioid withdrawal symptoms. To address this issue, the following had to be determined: (i) the lowest dose ("threshold dose") of Compound I that produces antagonism of opioid receptors in the CNS; and (ii) the doses of Compound I that would be expected to produce sufficient levels of antagonism of opioid receptors in peripheral tissues to be efficacious for treating OIC or OBD.

### Calculation of the lowest dose of Compound I that produces antagonism of opioid receptors in the CNS

Determination of the lowest dose of Compound I that produces antagonism of opioid receptors in the CNS was addressed directly by examining the reported effect of Compound I on pharmacodynamic markers of central opioid receptor antagonism that were measured in the single and multiple dose Phase 1 clinical studies, *i.e.,* the reversal of morphine-induced miosis (the effect on pupil diameter) and increases in adrenocorticotropic hormone (ACTH) and cortisol. The observed whole body dose of Compound I associated with a threshold reversal of morphine-induced miosis was 10 mg. Likewise, the observed whole body dose of Compound I associated with threshold increases in ACTH and cortisol was between 10 and 25 mg. A conservative estimate of the whole body dose for central opioid receptor antagonism was then determined by reducing these observed threshold doses by a half-log factor (*i.e.,* 3-fold). Thus, the minimum doses of Compound I associated with pharmacologically relevant antagonism of opioid receptors in the CNS were conservatively estimated to be in the range of from about 3 mg to about 10 mg.

### Calculation of dose of Compound I that would be expected to produce antagonism of opioid receptors in peripheral tissues sufficient to be efficacious for treating OIC or OBD

There are a number of approaches to determining the effective dose of Compound I for treating OIC or OBD. Four are outlined below. Depending on the approach, a number of basic assumptions were made in calculating the effective dose of Compound I for treating OIC or OBD: (i) The µ-opioid receptor is the primary target of Compound I to produce efficacy in the treatment of OIC or OBD, although other opioid receptors may also play a role. (ii) The systemic presence of Compound I in the periphery (but not in the CNS) is sufficient to produce robust efficacy in treating OIC or OBD. Thus, calculations were based on systemic plasma concentrations as opposed to possible topical or local effects in gut tissues, though these may also contribute to efficacy. (iii) The pharmacokinetic properties, specifically the oral bioavailability, of Compound I will be roughly similar in healthy volunteers and in patients with OIC or OBD. (iv) The pharmacokinetic parameters (in particular Cₘₐₓ, half-life (t_{1/2}) and area under the curve (AUC) for Compound I continue to be dose-proportional over the descending dose range of approximately 1 mg to 0.05 mg. Assumptions (iii) and (iv) were reasonable because Compound I was shown to have highly consistent and dose-proportional oral pharmacokinetics in humans over the dose range 1 mg to 100 mg. Moreover, subsequent experiments showed that Compound I has approximately 100% oral bioavailability in the cynomologus monkey over the dose range 0.1 mg/kg to 0.5 mg/kg.

The first approach was a calculation based on comparison of pharmacokinetic and pharmacological data for the highly peripheralized opioid receptor antagonist alvimopan. Specifically, pharmacokinetic and pharmacological data determined for Compound I were compared with the pharmacokinetic and pharmacological data for alvimopan measured in Phase 2 efficacy studies for treating patients with OIC or OBD. Webster et al. (2008) Pain 137:428-440. The question to be answered was what dose of Compound I would produce comparable systemic levels of µ-opioid receptor antagonism in the periphery to alvimopan, which is a function of the two drugs' affinity (Kᵢ) for the µ-opioid receptor, drug concentrations (assessed by Cₘₐₓ values) in the plasma, and extent of plasma protein binding. The Kᵢs of alvimopan and Compound I were determined by *in vitro* receptor binding in CHO cell membranes expressing cloned human µ-opioid receptors. As shown in Table 1, the µ-opioid receptor Kᵢ of alvimopan was 0.27 nM and the µ-opioid receptor Kᵢ of Compound I was 0.36 nM. The mean free alvimopan Cₘₐₓ plasma level following the efficacious dose of 0.5 mg BID alvimopan to patients suffering from OIC or OBD in Phase 2 clinical studies was measured as approximately 0.1 ng/ml. The mean free Cₘₐₓ plasma level following a 1 mg single oral dose of Compound I to healthy subjects was measured as 1.15 ng/ml. For these initial calculations, free drug was estimated using mouse plasma protein binding as a surrogate for human plasma protein binding; subsequent experimental data confirmed that these values were indeed comparable (*see* Table 1).

**Table 1**

| **Pharmacological and Pharmacokinetic data of alvimopan and compound I** | | |
|---|---|---|
| | Alvimopan | Compound I |
| µ-Opioid Receptor Kᵢ (nM) | 0.27 | 0.36 |
| Mean free Cₘₐₓ plasma level (ng/ml) | 0.1¹ | 1.15² |
| Plasma protein binding (% free drug) | 20.3 (human) | 64³ (mouse) |
| | | 73.8 (human) |

| | | |
|---|---|---|
| ¹Following 0.5 mg BID. (CI 0.03-0.25) ²Following 1 mg dose. (CI 1.66-2.16) ³Initial calculations using mouse data were made presuming human values would be comparable. | | |

Phase 1 studies showed that Compound I has an approximately linear relationship between dose and plasma Cₘₐₓ and area under the curve (AUC) over the dose range from 1 to 100 mg. The dose of Compound I needed to achieve Cₘₐₓ equal to 0.1 ng/ml (*i.e.,* the free plasma concentration of alvimopan effective for treating OIC or OBD) can therefore be estimated by linear extrapolation to lower doses, *i.e.,* assuming a linear relationship between dose and Cₘₐₓ at doses less than 1 mg. Using this approach, the dose of Compound I needed to produce a free drug plasma Cₘₐₓ of 0.1 ng/ml is 0.087 mg. The relative Kᵢs of the two compounds (Table 1) were factored in to determine the dose of Compound I required to achieve pharmacologically equivalent levels of µ-opioid receptor antagonism to those associated with the clinically efficacious dose of alvimopan. By simple proportion, this indicates a surprisingly low dose of 0.12 mg Compound I would be efficacious in treating OIC or OBD. Importantly, this dose is 25- to 83-fold lower than doses associated with antagonism of central opioid receptors, clearly indicating that, though centrally active at higher doses, Compound I is still sufficiently peripheralized to treat OIC or OBD at very low doses without compromising central opioid analgesia or precipitating central opioid withdrawal.

A second approach was a calculation based on the potency (Kᵢ) of Compound I as an antagonist for the µ-opioid receptor. In this case, pharmacokinetic and pharmacological data for Compound I were used to determine the dose of the compound that would produce a systemic plasma free drug concentration (Cₘₐₓ) in the periphery equal to the Kᵢ for the µ-opioid receptor (*i.e.,* 0.36 nM as determined by *in vitro* human µ-opioid receptor binding in CHO cells - Table 1). As discussed above, the mean free Cₘₐₓ plasma level following a 1 mg single oral dose of Compound I to healthy subjects was measured as 1.15 ng/ml, or 2.98 nM (molecular weight of Compound I: 386.4 g/mol). Assuming a linear relationship between dose and Cₘₐₓ for doses below 1 mg, the dose of Compound I needed to achieve 0.36 nM of free drug was calculated to be the surprisingly low 0.12 mg whole body dose. Again, this dose is 25- to 83-fold lower than doses associated with antagonism of central opioid receptors, likewise indicating that, though centrally active at higher doses, Compound I is still sufficiently peripheralized to treat OIC or OBD at very low doses without compromising central opioid analgesia or precipitating central opioid withdrawal.

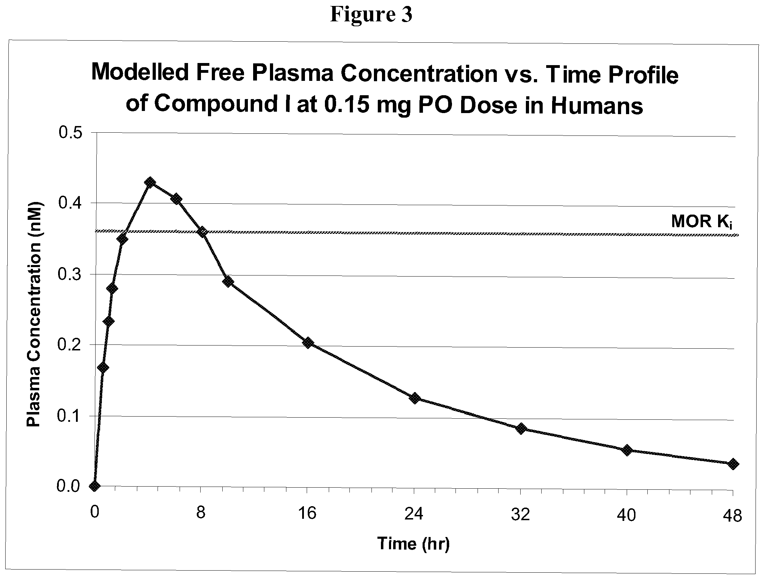

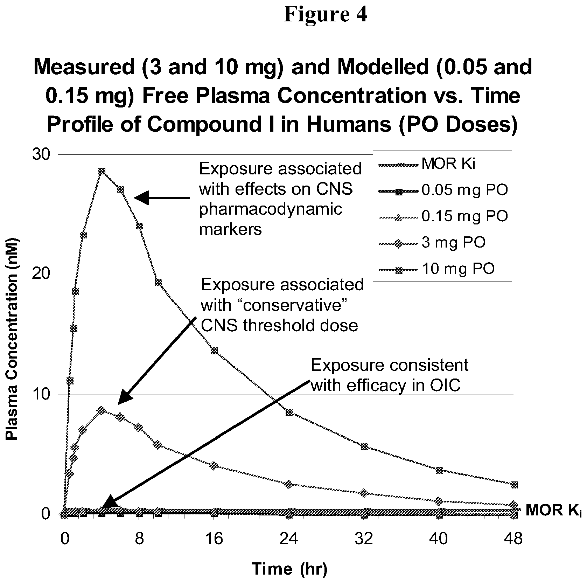

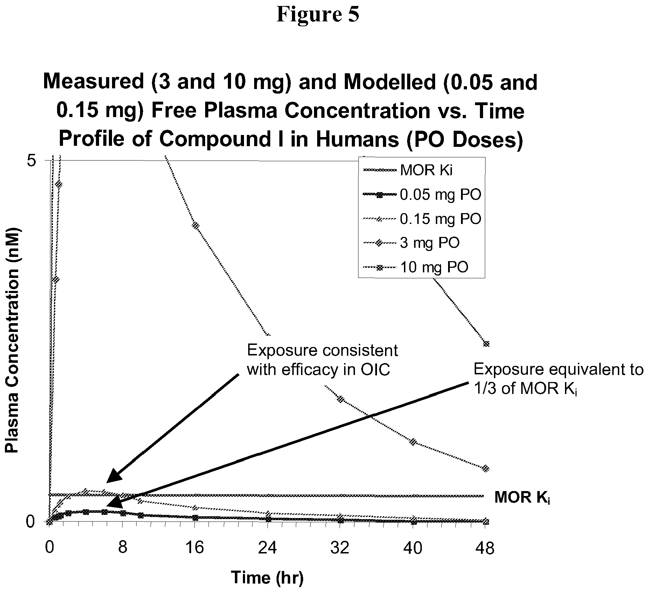

A third approach was to generate a pharmacokinetic model for Compound I at low doses. This was accomplished by assuming a linear relationship between dose and all parameters of the plasma time course for the 1 mg dose of Compound I, and extrapolating these to lower doses. This model shows that, following a dose of 0.15 mg of Compound I to humans, free plasma concentrations of the compound were at or above the µ-opioid receptor Kᵢ (0.36 nM) for about 6 hours (Figure 3), *i.e.,* sufficient time to produce an efficacy signal. This dose is 20- to 67-fold lower than doses associated with antagonism of central opioid receptors, again indicating that, though centrally active at higher doses, Compound I is sufficiently peripheralized to treat OIC or OBD at very low doses without compromising central opioid analgesia or precipitating central opioid withdrawal (Figure 4 and Figure 5).

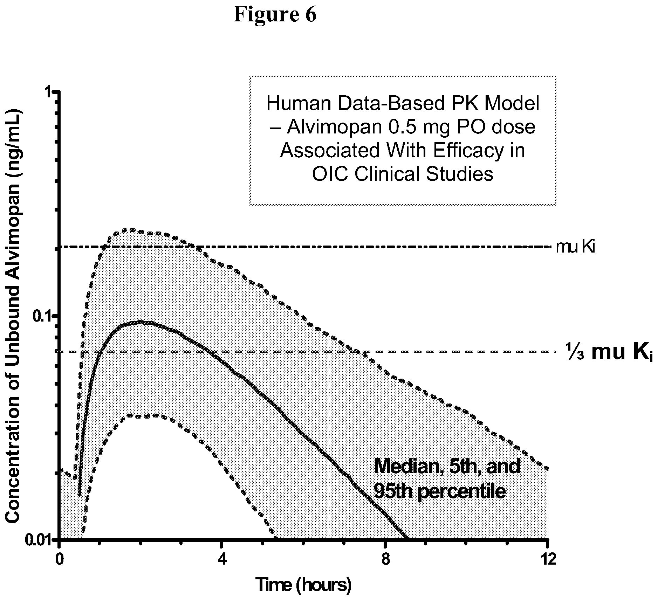

A fourth approach was to evaluate a clinical pharmacokinetic model for alvimopan that suggests a period of exposure to free drug concentrations that are only approximately 0.33 of the Kᵢ for the µ-opioid receptor may be sufficient to produce efficacy in OIC patients. (Figure 6) Using the pharmacokinetic model and assumptions described above, a dose of 0.5 mg Compound I would result in free plasma concentrations at or above 0.33 of the µ-opioid receptor Kᵢ (0.36 nM) for greater than 4 hours, *i.e.,* sufficient time to produce an efficacy signal. This dose is 60- to 200-fold lower than doses associated with antagonism of central opioid receptors, again indicating that, though centrally active at higher doses, Compound I is still sufficiently peripheralized to treat OIC or OBD without compromising central opioid analgesia or precipitating central opioid withdrawal.

At doses of 1-3 mg, Compound I has a measured plasma half-life in humans of approximately 12 hours. If there is a potential for a degree of drug accumulation upon repeated dosing regimens, the doses associated with efficacy for treating OIC or OBD may be appreciably lower than the doses calculated above, *i.e.,* as low as 0.01 to 0.02 mg whole body dose QD or BID (for a human male weighing approximately 70 kg).

### Example 3: Phase I multiple ascending dose clinical study conducted with Compound I; preliminary results

The objective of the Phase I multiple ascending dose clinical study was to evaluate the safety, tolerability, pharmacokinetics, and clinical effect of multiple ascending doses of Compound I administered twice daily (BID) to subjects who have opioid induced constipation (OIC) as a result of chronic opioid therapy for persistent noncancer pain. The first part of the study was a randomized, double blind, placebo controlled, multiple ascending dose study during which subjects took 4 oral doses of study medication over 2 days while confined to the study unit. Each of the 0.10 mg, 0.25 mg, 0.35 mg, 0.50 mg and 0.75 mg BID dose cohorts included 4 subjects, and randomization was unbalanced, with a 3:1 ratio of Compound I treatment (n=3) to placebo treatment (n=1). The 0.75 mg BID cohort included only 2 subjects. Assessments of clinical effect included the following measures, which were assessed starting immediately after the first dose of study medication through discharge on day 3: (i) time to first bowel movement after the first dose of study medication; (ii) number of bowel movements; and (iii) bowel movement Comfort Scores, stool weight, and stool consistency as measured by the Bristol Stool Scale for each bowel movement that occurs while the subject was confined to the study unit.

For pharmacokinetic analyses, blood samples were collected from each subject on day 1 at approximately 1 hour before the first dose of study medication (time 0) and at each of the following times after the first dose of study medication: 15 min, 30 min, 60 min, 90 min, 2 h, 3 h, 4 h, 6 h, 8 h, 10 h, and 12 h. Figures 7A, 7B and 7C indicate the plasma levels of Compound I obtained after the first dose over the dosing interval of 12 h and for each of the 5 doses tested (0.10 mg, 0.25 mg, 0.35 mg, 0.50 mg and 0.75 mg BID). As shown in Figure 7A, all doses provided plasma concentrations that were above the MOR Kᵢ of Compound I [Kᵢ (MOR) = 0.36 nM], which is indicated by a dashed line. Only at the lowest dose (0.1 mg) did the concentration fall below the MOR Kᵢ before the end of the dosing interval at 12 hours after this first dose; and even at this dose, concentrations were above the MOR Kᵢ for almost 9 hours (*see* Figure 7C). Preliminary data indicate that the 0.10 mg and 0.25 mg doses were well-tolerated and produced the desired pharmacological effects.

## Claims

1. A compound of formula (I): 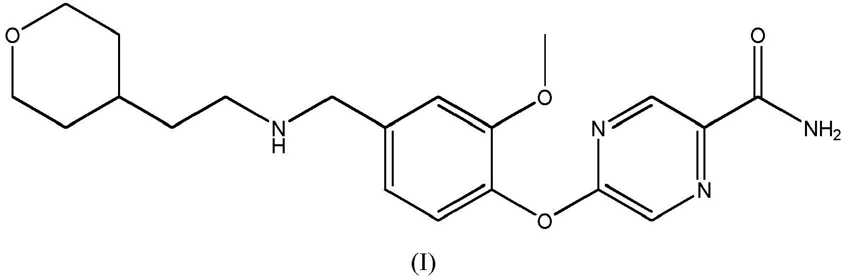 or a pharmaceutically acceptable salt thereof, for use in a method of treating or preventing a condition in a human subject associated with activation of opioid receptors in the periphery, comprising administering the compound to the subject in an amount ranging from about 0.01 mg to about 1.0 mg per day, wherein the condition is selected from the group consisting of postoperative ileus, postoperative nausea and vomiting, opioid-induced nausea and vomiting, opioid-induced respiratory depression, opioid-induced constipation, opioid-induced bowel dysfunction, chronic idiopathic constipation, constipation-predominant irritable bowel syndrome, intestinal pseudoobstruction, delayed gastric emptying, enteral feeding intolerance, narcotic ileus, obstipation, acceleration of gastrointestinal recovery following surgery and opioid-induced bloating.

2. The compound or pharmaceutically acceptable salt for use according to claim 1, wherein the compound is administered prior to or subsequent to or at the same time as an opioid analgesic.

3. The compound or pharmaceutically acceptable salt for use according to claim 2, wherein the condition is opioid-induced constipation.

4. The compound or pharmaceutically acceptable salt for use according to claim 2, wherein the condition is opioid-induced bowel dysfunction.

5. The compound or pharmaceutically acceptable salt for use according to any one of claims 1-4, wherein the compound of formula (I) or pharmaceutically acceptable salt administered to the subject in an amount of about 0.5 mg per day.

6. Use of a compound of formula (I): 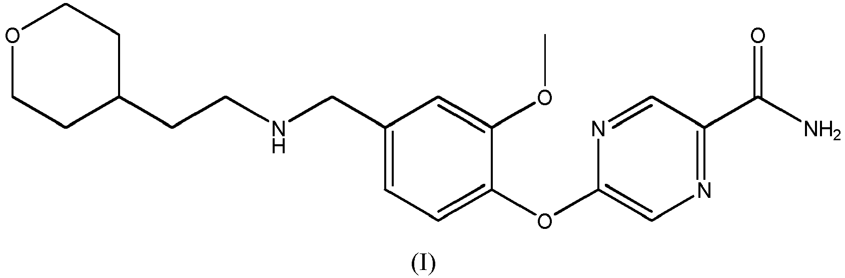 or a pharmaceutically acceptable salt thereof, for the manufacture of a medicament for treating or preventing a condition in a human subject associated with activation of opioid receptors in the periphery, comprising administering the compound to the subject in an amount ranging from about 0.01 mg to about 1.0 mg per day, wherein the condition is selected from the group consisting of postoperative ileus, postoperative nausea and vomiting, opioid-induced nausea and vomiting, opioid-induced respiratory depression, opioid-induced constipation, opioid-induced bowel dysfunction, chronic idiopathic constipation, constipation-predominant irritable bowel syndrome, intestinal pseudoobstruction, delayed gastric emptying, enteral feeding intolerance, narcotic ileus, obstipation, acceleration of gastrointestinal recovery following surgery and opioid-induced bloating.

7. The use according to claim 6, wherein the compound is administered prior to or subsequent to or at the same time as an opioid analgesic.

8. The use according to claim 6, wherein the condition is opioid-induced constipation.

9. The use according to claim 6, wherein the condition is opioid-induced bowel dysfunction.

10. The use according to any one of claims 6-9, wherein the compound of formula (I) or pharmaceutically acceptable salt administered to the subject in an amount of about 0.5 mg per day.

## Patentansprüche

1. Eine Verbindung der Formel (I): 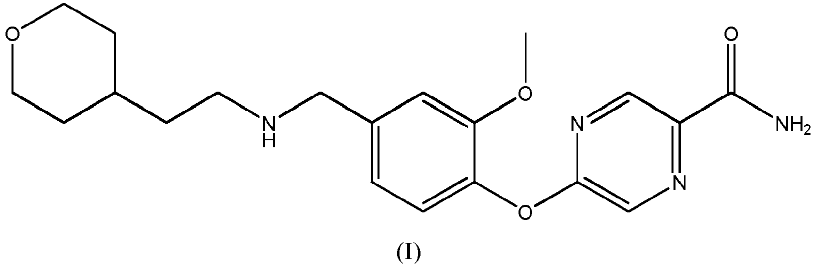 oder ein pharmazeutisch annehmbares Salz davon zur Verwendung bei einem Verfahren zur Behandlung oder Prävention eines mit der Aktivierung von Opioidrezeptoren in der Peripherie assoziierten Zustandes bei einem menschlichen Subjekt, umfassend die Verabreichung der Verbindung an das Subjekt in einer Menge, die von etwa 0,01 mg bis etwa 1,0 mg pro Tag reicht, wobei der Zustand ausgewählt ist aus der Gruppe bestehend aus postoperativem Ileus, postoperativer Übelkeit und postoperativem Brechreiz, opioidinduzierter Übelkeit und opioidinduziertem Brechreiz, opioidinduzierter Atemdepression, opioidinduzierter Konstipation, opioidinduzierter Darmstörung, chronischer idiopathischer Konstipation, konstipations-prädominantem Reizdarmsyndrom, intestinaler Pseudoobstruktion, verzögerter Entleerung des Magens, Unverträglichkeit von enteraler Ernährung, narkotischem Ileus, Obstipation, Beschleunigung der gastrointestinalen Genesung nach einem chirurgischen Eingriff, und opioidinduzierter Blähung.

2. Die Verbindung oder das pharmazeutisch annehmbare Salz zur Verwendung gemäß Anspruch 1, wobei die Verbindung vor oder nach oder gleichzeitig mit einem Opioid-Analgetikum verabreicht wird.

3. Die Verbindung oder das pharmazeutisch annehmbare Salz zur Verwendung gemäß Anspruch 2, wobei der Zustand opioidinduzierte Konstipation ist.

4. Die Verbindung oder das pharmazeutisch annehmbare Salz zur Verwendung gemäß Anspruch 2, wobei der Zustand opioidinduzierte Darmstörung ist.

5. Die Verbindung oder das pharmazeutisch annehmbare Salz zur Verwendung gemäß einem der Ansprüche 1-4, wobei die Verbindung der Formel (I) oder das pharmazeutisch annehmbare Salz in einer Menge von etwa 0,5 mg pro Tag an das Subjekt verabreicht wird.

6. Verwendung einer Verbindung der Formel (I): 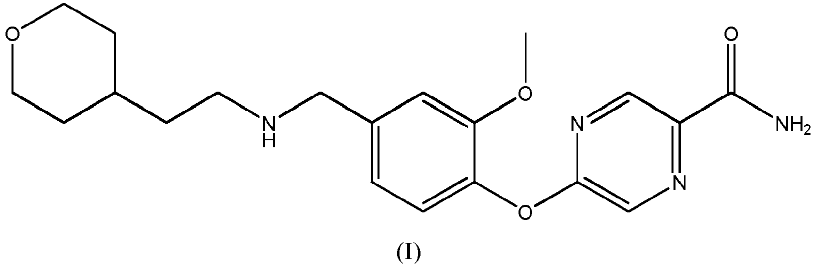 oder eines pharmazeutisch annehmbaren Salzes davon zur Herstellung eines Medikaments zur Behandlung oder Prävention eines mit der Aktivierung von Opioidrezeptoren in der Peripherie assoziierten Zustandes bei einem menschlichen Subjekt, umfassend die Verabreichung der Verbindung an das Subjekt in einer Menge, die von etwa 0,01 mg bis etwa 1,0 mg pro Tag reicht, wobei der Zustand ausgewählt ist aus der Gruppe bestehend aus postoperativem Ileus, postoperativer Übelkeit und postoperativem Brechreiz, opioidinduzierter Übelkeit und opioidinduziertem Brechreiz, opioidinduzierter Atemdepression, opioidinduzierter Konstipation, opioidinduzierter Darmstörung, chronischer idiopathischer Konstipation, konstipations-prädominantem Reizdarmsyndrom, intestinaler Pseudoobstruktion, verzögerter Entleerung des Magens, Unverträglichkeit von enteraler Ernährung, narkotischem Ileus, Obstipation, Beschleunigung der gastrointestinalen Genesung nach einem chirurgischen Eingriff, und opioidinduzierter Blähung.

7. Die Verwendung nach Anspruch 6, wobei die Verbindung vor oder nach oder gleichzeitig mit einem Opioid-Analgetikum verabreicht wird.

8. Die Verwendung gemäß Anspruch 6, wobei der Zustand opioidinduzierte Konstipation ist.

9. Die Verwendung gemäß Anspruch 6, wobei der Zustand opioidinduzierte Darmstörung ist.

10. Die Verwendung gemäß einem der Ansprüche 6-9, wobei die Verbindung der Formel (I) oder das pharmazeutisch annehmbare Salz in einer Menge von etwa 0,5 mg pro Tag an das Subjekt verabreicht wird.

## Revendications

1. Composé de la formule (I) : 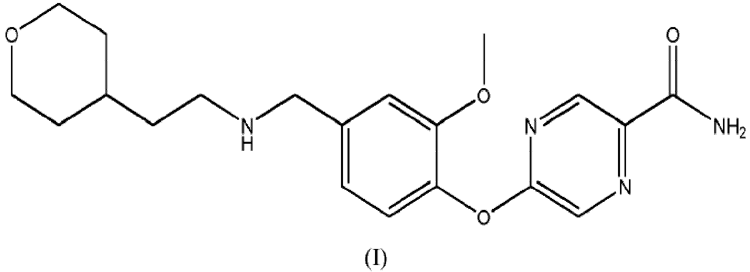 ou un sel pharmaceutiquement acceptable de celui-ci, à utiliser dans une méthode de traitement ou de prévention d'un état chez un sujet humain associé à l'activation des récepteurs opioïdes dans la périphérie, comprenant l'administration du composé au sujet en une quantité allant d'environ 0,01 mg à environ 1,0 mg par jour, où l'état est sélectionné parmi le groupe consistant en iléus postopératoire, nausée et vomissements postopératoires, nausée et vomissements induits par les opioïdes, dépression respiratoire induite par les opioïdes, constipation induite par les opioïdes, dysfonctionnement intestinal induit par les opioïdes, constipation idiopathique chronique, syndrome de l'intestin irritable à constipation prédominante, pseudo-obstruction intestinale, vidange gastrique retardée, intolérance à la nutrition entérale, iléus causé par narcotiques, obstipation, accélération du rétablissement gastro-intestinal suite à une intervention chirurgicale et ballonnement induit par les opioïdes.

2. Composé ou sel pharmaceutiquement acceptable à utiliser selon la revendication 1, où le composé est administré avant ou après ou en même temps qu'un analgésique opioïde.

3. Composé ou sel pharmaceutiquement acceptable à utiliser selon la revendication 2, où l'état est une constipation induite par les opioïdes.

4. Composé ou sel pharmaceutiquement acceptable à utiliser selon la revendication 2, où l'état est un dysfonctionnement intestinal induit par les opioïdes.

5. Composé ou sel pharmaceutiquement acceptable à utiliser selon l'une quelconque des revendications 1-4, où le composé de la formule (I) ou sel pharmaceutiquement acceptable est administré au sujet en une quantité d'environ 0,5 mg par jour.

6. Utilisation d'un composé de la formule (I) : 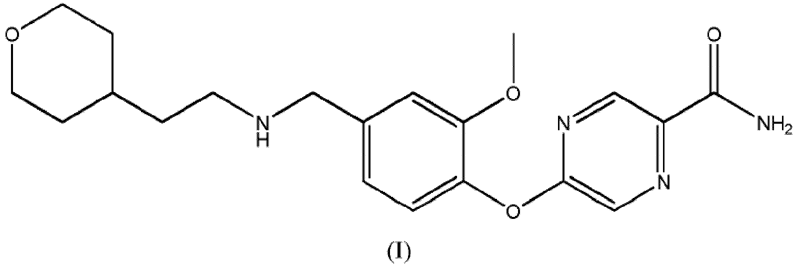 ou d'un sel pharmaceutiquement acceptable de celui-ci, dans la fabrication d'un médicament pour traiter ou prévenir un état chez un sujet humain associé à l'activation des récepteurs opioïdes dans la périphérie, comprenant l'administration du composé au sujet en une quantité allant d'environ 0,01 mg à environ 1,0 mg par jour, où l'état est sélectionné parmi le groupe consistant en iléus postopératoire, nausée et vomissements postopératoires, nausée et vomissements induits par les opioïdes, dépression respiratoire induite par les opioïdes, constipation induite par les opioïdes, dysfonctionnement intestinal induit par les opioïdes, constipation idiopathique chronique, syndrome de l'intestin irritable à constipation prédominante, pseudo-obstruction intestinale, vidange gastrique retardée, intolérance à la nutrition entérale, iléus causé par narcotiques, obstipation, accélération du rétablissement gastro-intestinal suite à une intervention chirurgicale et ballonnement induit par les opioïdes.

7. Utilisation selon la revendication 6, dans laquelle le composé est administré avant ou après ou en même temps qu'un analgésique opioïde.

8. Utilisation selon la revendication 6, dans laquelle l'état est une constipation induite par les opioïdes.

9. Utilisation selon la revendication 6, dans laquelle l'état est un dysfonctionnement intestinal induit par les opioïdes.

10. Utilisation selon l'une quelconque des revendications 6-9, dans laquelle le composé de la formule (I) ou sel pharmaceutiquement acceptable est administré au sujet en une quantité d'environ 0,5 mg par jour.
